# EUROPEAN PATENT APPLICATION

(11) **EP 4 775 983 A1**
(43) Date of publication of application: **15.07.2026**
(21) Application number: 25382007.0
(22) Date of filing: 09.01.2025
(51) Int. Cl.: G01N 33/18, G01N 27/40, G01N 27/416, G01N 17/00

(54) **SENSOR FOR DETECTING PHOSPHONATES AND MEASUREMENT SYSTEM USING SAID SENSOR**

(71) Applicant: Adiquimica S.A., 08024 Barcelona (ES)
(72) Inventor: Palacios Doñaque, Enric, 08490 BARCELONA (ES); Coma Salvans, Josep, 08024 BARCELONA (ES); Adroer Martori, Núria, 08024 BARCELONA (ES); Aumatell Colom, Jordi, 08024 BARCELONA (ES); Bosch Pons, Jaume, 08024 BARCELONA (ES); Faus Galtés, Oriol, 08024 BARCELONA (ES)
(74) Representative: Curell Suñol S.L.P.

(57) **Abstract**

Sensor (1) for detecting phosphonates comprising:
- a body (2) having a hollow vertical chamber (3), with a reference liquid (4) therein;
- a semipermeable membrane (5), arranged at the lower end of said chamber (3);
- two conductor wires (6,7), provided inside said chamber (3), in contact with said reference liquid (4) and separated from said membrane (5) at different distances;
- two output terminals (8, 9), each in electrical connection with one of said conductor wires (6, 7); and
- a first resistor (10) connected in parallel to said terminals (8, 9).

## Description

### Field of the invention

The invention relates to the field of detecting products in water, particularly, for detecting phosphonates forming antiscalant products, such as those used in desalination methods by means of reverse osmosis of seawater, permeate water, brackish water, and wastewater coming from tertiaries.

More specifically, the invention relates to a sensor for detecting phosphonates in water to be treated in order to be subject to a desalination process.

The invention also relates to an in-line measurement system for detecting phosphonates in water to be subjected to a desalination process comprising said sensor.

The invention also relates to the use of a semipermeable membrane for making a sensor for detecting phosphonates.

### State of the art

In desalination plants, antiscalants-dispersants are usually dosed in the feed water and, for example, in the inlet to the second reverse osmosis step for the removal of boron in seawater, brackish water, and permeate water. This latter process is performed at high pH values, for example between 10 and 11 and conversions of 75-80%. This can lead to precipitations, mainly, of calcium carbonate and magnesium hydroxide. To avoid this possible scaling of calcium and magnesium salts, antiscalant products are dosed.

Due to its composition, the analytical control of an antiscalant-dispersant is not always possible in a simple way. The antiscalants-dispersants that can be most easily analysed are those formulated based on phosphonates, since the analytical methods to determine their presence and/or concentration in water are based on the determination of organic phosphorus, and therefore, it is necessary to know the concentration of total phosphorus and phosphorus in the form of orthophosphate. The currently known methods are based on photometric determinations: the mathematical difference between the two concentrations corresponds to the concentration of organic phosphorus which, in turn, is equivalent to a certain concentration of antiscalant-dispersant product. Moreover, the determination of total phosphorus and orthophosphate needs a laboratory method where it is required prior treatment of the sample by boiling and hydrolysis and the subsequent application of a spectrophotometric analytical method with colour development proportional to the phosphorus concentration. Although the mentioned analytical methods can be automated, they are very costly to perform and there is always the need for reagent replenishment and very thorough maintenance. Furthermore, the frequency of analysis is very low, since the known analytical methods for determining the total phosphorus and orthophosphate require a preparation time of the sample and a response time for the reaction of phosphorus with the reagents indicated by the analytical method.

Therefore, a solution is needed that facilitates the analysis, that ideally can be used continuously, and that at the same time is reliable and has low manufacturing and maintenance costs.

### Description of the invention

The purpose of the invention is to provide a sensor for detecting phosphonates of the type indicated above, which allows the problems previously set forth to be solved.

This purpose is achieved by means of a sensor for detecting phosphonates of the type indicated above, characterized in that it comprises:
- a body, having a hollow vertical chamber, said chamber having an upper end and a lower end;
- a reference liquid, provided inside said chamber;
- a semipermeable membrane arranged at said lower end of said chamber, interposed separating the outside of said chamber and the inside of said chamber; said membrane having an outer face, facing said outside of said chamber, and an inner face, facing said inside of said chamber;
- a first conductor wire, arranged inside said chamber, in contact with said reference liquid and separated a first distance from said inner face of said membrane, preferably around 3 mm;
- a second conductor wire, arranged inside said chamber, in contact with said reference liquid and separated a second distance from said inner face of said membrane, said second distance being greater than said first distance;
- a first output terminal in electrical connection with said first conductor wire;
- a second output terminal in electrical connection with said second conductor wire; and
- a first resistor connected in parallel between said first output terminal and said second output terminal.

The sensor described is based on the development of the potential that is generated in the semipermeable membrane due to the gradient of the phosphonate concentration between the p outside the membrane (which, in use, is submerged in water), and the phosphonate concentration in the reference liquid housed inside the body of the sensor. The gradient of concentrations is established in any part of the membrane. The developed potential generates a current running through the first conductor wire. The current generated is transmitted through the internal phosphonate solution which is highly conductive. The first resistor is arranged in parallel between the two output terminals, and at the same time in series between the first conductor wire and the second output terminal. In that sense, a potential drop according to Ohm's law, which can be measured between the output terminals, takes place between the connectors of the first resistor. Particularly advantageous, a measurement of the potential difference with an external reference electrode is not necessary with the described configuration, unlike with other selective sensors (for example, pH sensors) where it is necessary to use an external reference electrode, for example, Ag/AgCI, LiCI, or calomel electrodes.

This type of sensor allows to be continuously used, which is typically referred to as "in-line", i.e., the sensor is used to detect the compounds as water circulates through the system, without the need for an analysis based on samples or the like. This type of solution is therefore more suitable to be used in the workplace. Furthermore, given the structural simplicity of the sensor and its components, it is possible to keep manufacturing costs low, resulting in a low cost of the end product.

Preferably, the sensor further comprises a second resistor provided in series between said second conductor wire and said second terminal. The second resistor closes the circuit, limiting the electrical closing current.

Preferably, said reference liquid comprises distilled water with a known concentration of the product to be detected, particularly a phosphonate-based antiscalant substance, such that the sensor can be used for detecting this type of components in seawater, brackish water, water coming from permeate or from tertiary treatments. It is possible to foresee other applications, in which the liquid and/or the substances to be detected are different.

Preferably, said membrane is made of at least one of:
- cellulose acetate-nitrate;
- polyvinyl chloride, PVC;
- polysulfone, PSF;
- polysulfone supported on nonwoven fabric; and
- polycarbonate.

By way of illustrative and non-limiting example, during the development phase of the invention, the membranes were made by dissolving an amount of the corresponding polymer in tetrahydrofuran (THF) by stirring the mixture, although other organic solvents can be envisaged based on the material used to make the membrane. The solvent (THF) was left to evaporate in a heater at 40°C and in a controlled manner, for 48 hours. To achieve disc-shaped membranes, the polymer and THF mixture was poured into a cylindrical glass mould supported on a preferably polished glass plate and the assembly was introduced into the heater. Once the indicated time elapsed, the membranes were removed from the mould, and they had said shape and thicknesses comprised between 35 and 42 µm. Once the membranes were obtained, they were characterized by performing water permeability and specific resistance tests. It was thereby observed that the membranes having better permeability and specific low resistance were the membranes made of cellulose acetate and nitrate and those made of polysulfone, and thus, these are preferred embodiments, particularly for seawater desalination environments, in which the presence of phosphonates is to be detected.

Preferably, said chamber has a cylindrical shape, which is open at said lower end, such that said membrane occupies all said lower end of said chamber. The contact of the membrane with the external medium in which phosphonates are to be detected is thereby maximized.

In an alternative embodiment, said chamber has a cylindrical shape with an opening at said lower end, which does not occupy all said lower end, and in which said membrane is arranged. This option decreases exposure to the external medium, which causes the concentration density of the substance to be detected, particularly phosphonates, in the external solution to increase on the outer face of the membrane.

Preferably, said first conductor wire and said second conductor wire are each made of a corrosion-resistant material, such that they can be exposed to the reference liquid, which can generally be corrosive, for example, distilled water with a known phosphonate concentration. The material is preferably silver-palladium, with a good performance for such environments.

The invention also relates to the use of a semipermeable membrane for making a sensor suitable for detecting phosphonates, being a diffusion sensor type, according to one of the embodiments described above, of the type allowing the diffusion potential of the concentrations of a substance of the inner reference liquid, for example, phosphonates, and the external medium of the sensor, to be measured.

The invention also relates to an in-line measurement system for detecting phosphonates in water to be subjected to a desalination process, having:
- a channel configured to circulate water therein, having a water inlet and a water outlet; and
- a sensor according to any one of the embodiments described above;
wherein said body of said sensor is introduced in said channel, such that said outer face of said semipermeable membrane is arranged such that it is in contact with said water circulating through said channel.

The sensor is thereby exposed to the liquid in which a specific substance is to be detected, and this measurement can be taken even in real time, while the liquid circulates through the system. A non-excluding example of the application of this system is the real-time measurement of the phosphonate concentration at the inlet to a second step of reverse osmosis for seawater desalination, in which a phosphonate-based antiscalant has been added. The output signal of the sensor can then be displayed and/or stored in a memory for the subsequent consultation thereof. Thus, control means configured to perform the process of obtaining the measurements of said sensor, the display and storage thereof, can be provided. It can further be envisaged that said control means obtain concentration values that are determined based on the potential difference measurements obtained by the sensor. Said concentration data can be determined, for example, based on a pre-calibration test based on the exposure of the sensor to known concentration values for the substance to be measured, even for different temperatures, pH, and/or flow rate of the liquid.

Preferably, the system further comprises a temperature sensor configured to measure the temperature inside said water circulating through said channel, which allows the potential difference values obtained by the sensor based on said temperature to be corrected. The correction data may have previously been obtained experimentally, as described above.

Preferably, said system further comprises water flow selection means in fluid connection with said water inlet of said channel, said water flow selection means being configured to alternate between a first reference water flow, particularly, water to which no antiscalants have been added during a desalination process, and a second water flow in which the concentration of a substance is to be detected, particularly the phosphonate concentration due to the antiscalant added for a desalination process.

It can thereby be envisaged that the first reference water flow (for example, without antiscalant) is used to establish the baseline and to condition the sensor during a first programmed time interval. The second water flow, with the water to be measured (for example, with antiscalant), is used for the measurement itself during another programmed time interval.

Preferably, the system further has control means, configured to control said water flow selection means according to a predefined sequence, such that it is possible to alternate between said first reference water flow and said second water flow to be measured. This prevents saturation of the sensor and allows the use thereof for longer times.

Preferably, said water flow selection means comprise a first valve for said first water flow and a second valve for said second water flow. In that sense, to perform the function of switching between the first and the second flow, two valves, for example, valves of regulation electronic are used. Additionally, it can be envisaged that in addition to cutting off the passage of water flow, said valves serve to regulate the flow rate thereof, such that precise control of the measurement and conditioning periods can be obtained.

The invention also comprises other detail features illustrated in the detailed description of an embodiment of the invention and in the attached figures.

### Brief description of the drawings

The advantages and features of the invention will become apparent from the following description in which, without a non-limiting character with respect to the scope of the main claim, preferred embodiments of the invention are set forth in reference to the figures.
Figure 1 is a schematic representation of a sensor according to an embodiment of the invention.
Figure 2 is a schematic and conceptual representation of the operating elements on which the sensor of the invention is based.
Figure 3 is a detail schematic representation of the use of the sensor of the invention in an in-line configuration in a continuous detection system.
Figure 4 is a schematic representation of an in-line measurement system according to an embodiment of the invention.

### Detailed description of embodiments of the invention

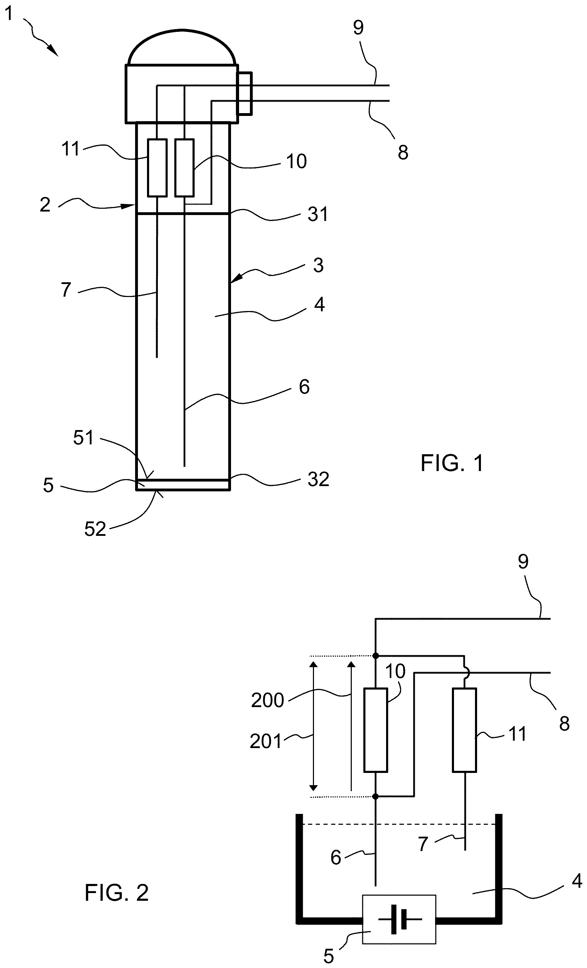

Figure 1 shows a first embodiment of a sensor 1 suitable for detecting phosphonates in water to be subjected to a desalination process. The sensor has a body 2 having a hollow vertical chamber 3 with an upper end 31 and a lower end 32. The chamber 3 is configured to house inside a reference liquid 4 which, for the first embodiment, is distilled water with a known phosphonate concentration. In the first embodiment, the chamber 3 is cylindrical and the body 2 has a housing portion for electronics in the upper portion thereof, which is separated from the chamber 3 such that the latter is separated from the reference liquid 4.

A semipermeable membrane 5 is arranged at the lower end 32 of chamber 3, such that it is interposed between the outside and the inside of said chamber 3. In the case of the first embodiment, membrane 5 is made of cellulose acetate-nitrate. Membrane 5 has an outer face 52, facing the outside, and an inner face 51, facing the inside of said chamber 3. As can be seen in Figure 1, in the first embodiment membrane 5 occupies all said lower end 32 of chamber 3. When sensor 1 is in use, the outer face 52 of the membrane is exposed to the medium, in which the presence of a substance to be detected is to be measured, which, in the case of the first example, are phosphonates of antiscalant products.

A first conductor wire 6 and a second conductor wire 7 are arranged inside chamber 3, and therefore, they are submerged in reference liquid 4, so that these conductor wires are made of a corrosion-resistant material which, in the first embodiment, is silver-palladium. Both conductor wires 6,7 extend vertically to the upper end 31 of the chamber, without actually touching membrane 5 on the inner face 51 thereof. Furthermore, the distance between membrane 5 and the second conductor wire 7 is greater than the distance between the membrane and the first conductor wire 6. Particularly, for the first embodiment, the distance between the inner face 51 of the membrane 5 and the first conductor wire 6 is 3 mm, whereas the distance between said inner face 51 and the second conductor wire 7 is 40 mm. Due to diffusion effect, an electric current is generated between both conductor wires that depends on the concentrations of the inner reference liquid 4 and the external media. This has conceptually been shown in Figure 2, where membrane 5 is shown as the equivalent to an energy source. To obtain the measurement, sensor 1 has a first resistor 10, which is electrically connected between the two conductor wires 6, 7; in the first embodiment, the first resistor 10 has a value of 10 kΩ. An electric current 200 thereby circulates through said first resistor 10, and an electric potential difference 201 according to the Ohm's law, V=I·R, is established at the ends thereof. Each of the ends of the first resistor 10 is connected to an output terminal 8, 9: a first terminal 8 connected to the end in direct electrical connection with the first conductor wire 6, and a second terminal 9 connected to the opposite end of the resistor, such that the first resistor 10 is connected in parallel between said output terminals 8, 9. The second terminal 9 is, in turn, in electrical connection with the second conductor wire 7, in the case of the first embodiment, through a second resistor 11 connected in series to close the circuit, limiting the electrical closing current. In the described embodiment, the second resistor 11 has a value of 4.7 kΩ.

In the first embodiment, body 2 of sensor 1 has an approximate length of 140 mm, 100 mm of which correspond to the tube containing chamber 3 and 40 mm to the upper head. The outer diameter of chamber 3 is 12 mm and the inner diameter is 8 mm. Membrane 5 has a diameter of 12 mm, and thus, it completely covers the hollow inner diameter of chamber 3 and is attached to body 2 by means of a tail.

Other embodiments of the invention sharing most of the features of the first embodiment described above are described below. Accordingly, hereinafter only the differentiating elements will be described. The same reference numbers have been used in the figures as in the first embodiment to designate equivalent elements.

In a second embodiment not shown in the figures, chamber 3 has a cylindrical shape with an opening at said lower end 32 which does not occupy all said lower end 32 and in which said membrane 5 is arranged.

In a third embodiment, membrane 5 is made of polysulfone, PSF.

In a fourth embodiment, membrane 5 is made of polysulfone supported on nonwoven fabric.

In a fifth embodiment, membrane 5 is made of polyvinyl chloride, PVC.

In a sixth embodiment, membrane 5 is made of polycarbonate.

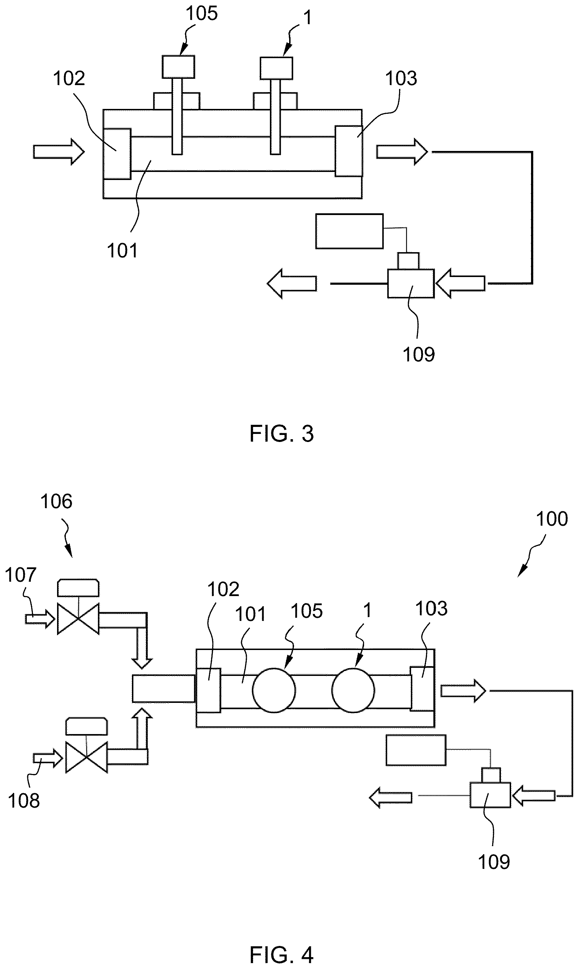

Figure 3 and Figure 4 show a first embodiment of an in-line measurement system 100 for detecting phosphonates in water to be subjected to a desalination process. As can be seen in the detail of Figure 3, system 100 has a channel 101 through which water, in which phosphonates are to be measured, is circulated. Channel 101 has a water inlet 102 and a water outlet 103. The water flow has schematically been shown with arrows in the figures.

A sensor 1 according to one of the embodiments described above is arranged such that the body 2 of sensor 1 is introduced in said channel 101, so the outer face 52 of the semipermeable membrane 5 of sensor 1 is in contact with the water circulating through channel 101. In this embodiment, system 100 is further provided with a temperature sensor 105 which is arranged in channel 101 to measure the temperature of the water circulating inside said channel 101, as well as with a flow rate sensor 109, downstream of water outlet 103 of channel 101.

Thus, it is an in-line configuration and system 100 allows to take continuous measurements of the phosphonates present in the water by means of sensor 1.

Likewise, system 100 further comprises water flow selection means 106, which are in fluid connection with water inlet 102 of channel 101. These water flow selection means 106, comprising a first valve for controlling first water flow 107 and a second valve for controlling second water flow 108, are configured to alternate between a first reference water flow 107 and a second water flow 108, in which a substance is to be detected. This alternation allows the cleaning and recalibration of the system 100, for example, according to a predefined sequence. By way of a non-limiting example, a first water flow 107 not containing antiscalants is circulated, such that the system can be measured and calibrated with respect to that reference. Subsequently, the first water flow 107 is cut off and it is circulated the second flow 108 corresponding to the same type of water but to which antiscalants have been added.

## Claims

1. A sensor (1) suitable for detecting phosphonates in water to be subjected to a desalination process, **characterized in that** it comprises:
- a body (2) having a hollow vertical chamber (3), said chamber (3) having an upper end (31) and a lower end (32);
- a reference liquid (4) provided inside said chamber (3);
- a semipermeable membrane (5), arranged at said lower end (32) of said chamber (3), interposed separating the outside of said chamber (3) and the inside of said chamber (3); said membrane (5) having an outer face (52), facing said outside of said chamber (3), and an inner face (51), facing said inside of said chamber (3);
- a first conductor wire (6), arranged inside said chamber (3), in contact with said reference liquid (4) and separated a first distance from said inner face (51) of said membrane (5);
- a second conductor wire (7), arranged inside said chamber (3), in contact with said reference liquid (4) and separated a second distance from said inner face (51) of said membrane (5), said second distance being greater than said first distance;
- a first output terminal (8) in electrical connection with said first conductor wire (6);
- a second output terminal (9) in electrical connection with said second conductor wire (7); and
- a first resistor (10) connected in parallel between said first output terminal (8) and said second output terminal (9).

2. Sensor (1) according to claim 1, further comprising a second resistor (11), provided in series between said second conductor wire (7) and said second terminal (9).

3. Sensor (1) according to any one of claims 1 or 2, wherein said aqueous medium comprises distilled water and a known concentration of a product to be detected, preferably a phosphonate-based antiscalant substance.

4. Sensor (1) according to any one of claims 1 to 3, wherein said membrane (5) is made of at least one of:
- cellulose acetate-nitrate;
- polyvinyl chloride, PVC;
- polysulfone, PSF;
- polysulfone supported on nonwoven fabric; and
- polycarbonate;
preferably cellulose acetate-nitrate and/or PSF.

5. Sensor (1) according to any one of claims 1 to 4, wherein said chamber (3) has a cylindrical shape, which is open at said lower end (32), such that said membrane (5) occupies all said lower end (32) of said chamber (3).

6. Sensor (1) according to any one of claims 1 to 4, wherein said chamber (3) has a cylindrical shape with an opening at said lower end (32) which does not occupy all said lower end (32), and wherein said membrane (5) is arranged.

7. Sensor (1) according to any of claims 1 to 6, wherein said first conductor wire (6) and said second conductor wire (7) are each made of a corrosion-resistant material, preferably, silver-palladium.

8. Use of a semipermeable membrane (5) for making a sensor (1) according to any one of claims 1 to 7, being a diffusion sensor type.

9. In-line measurement system (100) for detecting phosphonates in water to be subjected to a desalination process, having:
- a channel (101), configured to circulate water therein, having a water inlet (102) and a water outlet (103); and
- a sensor (1) according to any one of claims 1 to 7;
wherein said body (2) of said sensor (1) is introduced in said channel (101), such that said outer face (52) of said semipermeable membrane (5) is arranged, so that it is in contact with said water circulating through said channel (101).

10. System (100) according to claim 9, further comprising a temperature sensor (105), configured to measure the temperature inside said water circulating through said channel (101).

11. System (100) according to any one of claims 9 or 10, further comprising water flow selection means (106) in fluid connection with said water inlet (102) of said channel (101), said water flow selection means (106) being configured to alternate between a first reference water flow (107) and a second water flow (108) in which a substance is to be detected.

12. System (100) according to claim 11, further having control means, configured to control said water flow selection means (106) according to a predefined sequence.

13. System (100) according to any one of claims 11 or 12, wherein said water flow selection means (106) comprise a first valve for said first water flow (107) and a second valve for said second water flow (108).
